# EUROPEAN PATENT SPECIFICATION

(11) **EP 1 878 418 B1**
(45) Date of publication and mention of the grant of the patent: **07.10.2009**
(21) Application number: 07013289.9
(22) Date of filing: 06.07.2007
(51) Int. Cl.: A61K 6/00, A61K 6/083

(54) **Dental Cement**
Zahnzement
Ciment dentaire

(30) Priority: 11.07.2006 JP 2006190466
(43) Date of publication of application: 16.01.2008
(73) Proprietor: GC Corporation, Tokyo 174-8585 (JP)
(72) Inventor: Tokui, Hideki, Itabashi-ku Tokyo 174-8585 (JP); Yarimizu, Hideki, Itabashi-ku Tokyo 174-8585 (JP); Ota, Daisuke, Itabashi-ku Tokyo 174-8585 (JP); Mori, Takuya, Itabashi-ku Tokyo 174-8585 (JP); Nakaseko, Hisashi, Itabashi-ku Tokyo 174-8585 (JP)
(74) Representative: Müller-Boré & Partner Patentanwälte

(56) References cited:
- WO-A-20/04100900
- US-B1- 6 291 548
- US-B1- 6 369 164

## Description

The present invention relates to dental cement which is applied for dental repair and so on.

Conventionally, zinc phosphate cement, carboxylate cement, glass ionomer cement, and resin cement have been widely used for dental cement. Among them, the frequency of the use of zinc phosphate cement has been decreasing because of its non-adhesiveness to tooth dentin, low pH values due to phosphoric acid contained therein, possibility of occurrence of irritation to tooth structure at the initial process of curing thereof. Although carboxylate cement has low irritation to tooth structure, it is not reliable because of its low mechanical strength. Glass ionomer cement, which is a dental cement used by reacting polycarboxylic acid with fluoroaluminosilicate glass powder under the presence of water to cure, has been widely used in dental field since it has excellent characteristics, for example, it is extremely good in biocompatibility; the cured cement thereof is translucent and has excellent aesthetic appearance; it has excellent adhesiveness to tooth structure such as an enamel and a dentin; and it exhibits dental caries resistance by gradually releasing the fluoride ion contained in the fluoroaluminosilicate glass. However, the flexural strength thereof is lower than that of resin cement, resulting in being frangible. On the other hand, resin cement is excellent in mechanical strength but it has a defect of being non-adhesive to tooth structure.

Therefore, resin modified glass ionomer cement having a polymerizable monomer such as (meth)acrylate monomer as a resin component blended therein has been developed in order to solve problems of glass ionomer cement for dental use such as its frangibility, in particular, low flexural strength, in comparison with resin cement, and its high solubility in water after curing (refer to, for example, JP-A-08-026,925, JP-A-09-255,515, and JP-B-06-070,088).

However, such resin modified glass ionomer cement has been disadvantageous to have large hygroscopic expansion. The reason thereof is that the resin modified glass ionomer cement necessarily comprises a highly hydrophilic polymerizable monomer having hydroxyl groups and a molecular weight of less than 160, for example, 2-hydroxyethyl methacrylate, because it contains polycarboxylic acid, water, and polymerizable monomers being hardly soluble in water as a solution agent, while those should be dissolved to one another, and the polymerizable monomer such as 2-hydroxyethyl methacrylate exhibits extremely high hydrophilic property due to its molecular structure, resulting in that the cured cement thereof becomes to have characteristic that it absorbs water and expands within an oral cavity. Since such a cured cement in which hygroscopic expansion have occurred may break a dental prosthesis by the expansion stress of the cured cement, when a ceramic crown of low strength is used as the dental prosthesis, there still exists a problem that the conventional resin modified glass ionomer cement can not be applied to the ceramic crown of low strength.

The present inventors have proposed a dental cement using a solution agent comprising 4-methacryloxyethyl trimellitic acid and water instead of using polycarboxylic acid and 2-hydroxyethyl methacrylate or the like (refer to JP-A-2000-53,518). However, this dental cement has a high content of 4-methacryloxyethyl trimellitic acid having acid groups, which will produce a lot of salt comprising metallic ions originated from fluoroaluminosilicate glass powder or metal oxide powder comprising mainly zinc oxide as a powdery agent at the initial curing reaction, and the salt will be dissolved in an aqueous solution as time elapsed, resulting in providing a disadvantage that the cured cement exhibits high solubility in water.

An object of the present invention is to provide a dental cement that exhibits a high mechanical strength and adhesiveness to tooth structure as much as those of resin modified dental glass ionomer cement or resin cement and less hygroscopic expansion characteristic, and, further, that can solve the problem of the cured cement of high solubility in water.

Therefore, the present inventors have studied to solve the above mentioned problems and found a good composition comprising a first paste containing a (meth)acrylate monomer having an acid group, a (meth)acrylate monomer not having an acid group and having a specific molecular weight, a filler having a specific average particle size that is inactive to the (meth)acylate monomer having an acid group, and a polymerization accelerating agent for a polymerization catalyst in the second paste mentioned below; and a second paste comprising a (meth)acrylate monomer not having an acid group and having a specific molecular weight similar to the (meth)acrylate monomer not having an acid group and having a specific molecular weight in the first paste, a filler having a specific average particle size, and a polymerization catalyst for polymerizing the (meth)acrylate monomer having an acid group and the (meth)acrylate monomer not having an acid group, where the composition does not require to comprise a polymer having an acid group such as a polycarboxylic acid, 2-hydroxyethyl methacrylate or the like, thereby achieving the present invention that can solve the above mentioned problems.

In particular, the present invention provides a dental cement that comprises a first paste containing 5 to 75 % by weight of a (meth)acrylate monomer having an acid group, 5 to 55 % by weight of a (meth)acrylate monomer not having an acid group, having two or less of hydroxyl groups and/or amino groups and a having molecular weight of 160 or more, 10 to 80 % by weight of a filler being inactive to the (meth)acrylate monomer having an acid group and having an average particle size ranging from 0.05 to 20 µm, and 0.01 to 5 % by weight of an amine compound as a polymerization accelerator for a polymerization catalyst in a second paste described below; and
a second paste containing 10 to 75 % by weight of a (meth)acrylate monomer not having an acid group, having two or less of hydroxyl groups and/or amino groups, and having a molecular weight of 160 or more, 20 to 85 % by weight of a filler having an average particle size ranging from 0.05 to 20 µm, and 0.01 to 10 % by weight in total of an organic aromatic compound having at least one -SO₂- group and a peroxide as a polymerization catalyst for polymerizing the (meth)acrylate monomer having an acid group and the (meth)acrylate monomer not having an acid group, having two or less of hydroxyl groups and/or amino groups and having a molecular weight of 160 or more, the dental cement being used by mixing the first and second pastes in a weight ratio of the second paste to the first paste being in the range from 0.25 to 5.

In such dental cement, there are also an aspect in which the first paste further comprises 1 to 20 % by weight of water, and an aspect in which 0.1 to 10 % by weight of an inorganic thickening agent and/or an organic thickening agent having an average particle size ranging from 5 to 40 nm is contained in either of the first or second paste; and 0.01 to 3 % by weight of a photo polymerization catalyst for accelerating the polymerization of the (meth)acrylate monomer having an acid group and the (meth)acrylate monomer not having an acid group, having two or less of hydroxyl groups and/or amino groups and having a molecular weight of 160 or more is contained in either of the first or second paste.

Further, in each of these aspects, there are a case in which a monomer having carboxylic group is contained as the (meth).acrylate monomer having an acid group in the first paste and a case in which the filler having an average particle size ranging from 0.05 to 20 µm in the second paste is a filler being inactive to the (meth)acrylate monomer having an acid group in the first paste, or is metal oxide powder that can cause cement reaction (acid-base reaction) with the (meth)acrylate monomer having an acid group in the first paste under the presence of water to cure, and is used in fluoroaluminosilicate glass powder, dental zinc phosphate cement powder or dental carboxylate cement powder. When it is fluoroaluminosilicate that can cause this cement reaction to cure or the metal oxide powder, the rate of water in the mixture of the first and the second pastes is preferred to be in the range from 3 to 10 % by weight.

The dental cement in accordance with the present invention is excellent dental cement that has a high mechanical strength as much as the resin modified type dental glass ionomer cement or the resin cement while it exhibits adhesiveness to tooth structure and less hygroscopic expansion, and that can decrease the solubility in water.

The (meth)acrylate monomer having an acid group that is a component of the first paste of the dental cement in accordance with the present invention itself exhibits an effect to impart adhesiveness to tooth structure as well as polymerizes with the other component of the (meth)acrylate monomer not having an acid group and having a specific molecular weight to cure to form a matrix of dental cement. When the mixing quantity of this (meth)acrylate monomer having an acid group in the first paste is less than 5 % by weight, the adhesiveness of the dental cement is low, while when it exceeds 75 % by weight, the dental cement in accordance with the present invention will be expensive because the (meth)acrylate monomer having an acid group is expensive.

For the (meth)acrylate monomer having an acid group that is one component of the first paste of the dental cement in accordance with the present invention, it is preferred to be (meth)acrylate monomer having a phosphoric acid group or a carboxyl group as the acid group. Since the phosphoric acid group exhibits acidity stronger than that of the carboxylic group when water is present, it is highly effective to dissolve a smear layer on a tooth or to demineralize tooth structure, in particular, it exhibits high improvement effect of adhesiveness to an enamel. The polymerizable monomer having a phosphoric acid group is a polymerizable monomer having one or a plurality of phosphoric acid group in one molecule and examples thereof include 2-(meth)acryloyloxyethyl dihydrogen phosphate; bis(meth)acyloxyethyl phosphate; bis[2-(meth)acryloyloxyethyl]hydrogen phosphate; 2-(meth)acryloyloxyethyl phenylhydrogen phosphate; acid phosphoxyethyl(meth)acrylate; 6-(meth)acryloyloxyhexyl dihydrogen phosphate; 6-(meth)acryloyloxyhexyl phenylhydrogen phosphate; 10-(meth)acryloyloxydecyl dihydrogen phosphate; 1,3-di(meth)acryloylpropane-2-dihydrogen phosphate; 1,3-di(meth)acryloylpropane-2-phenylhydrogen phosphate; bis[5-{2-(meth)acryloyloxyethoxy carbonyl}]heptyl]hydrogen phosphate; and a reaction product of 6-hexanolide addition polymer of 2-hydroxyethyl(meth)acrylate with anhydrous phosphoric acid. Among them, 10-(meth)acryloyloxydecyl dihydrogen phosphate is particularly preferred in view of adhesiveness and stability of the monomer itself. These polymerizable monomers having the phosphoric acid group may be used alone or by mixing two or more of them.

For the monomer having the carboxyl group, examples thereof include 4-(meth)acryloxyethyl trimellitic acid; 4-(meth)acryloxyethyl trimellitic acid anhydride; 4-(meth)acryloxydecyl trimellitic acid; 4-(meth)acryloxydecyl trimellitic acid anhydride; 11-(meth)acryloyloxy-1,1-undecane dicarboxylic acid; 1,4-di(meth)acryloyloxy pyromellitic acid; 2-(meth)acryloyloxyethyl maleic acid; 2-(meth)acryloyloxyethyl phthalic acid; and 2-(meth)acryloyloxyethyl hexahydrophthalic acid. Among them, 4-(meth)acryloxyethyl trimellitic acid and 4-(meth)acryloxyethyl trimellitic acid anhydride are particularly preferred in view of adhesiveness.

When the monomer having the carboxyl group such as 4-(meth)acryloxyethyl trimellitic acid anhydride and the like is used as the (meth)acrylate monomer having an acid group, storing stability is improved by using it in the form of an aqueous solution. Therefore, the first paste of the cement is preferred to further comprise 1 to 20 % by weight of water. When the mixing quantity of water is less than 1 % by weight, it is difficult to attain the effect to improve the storing stability of the (meth)acrylate monomer having an acid group, while when it exceeds 20 % by weight, the resulting cured cement tends to be poor in mechanical strength, in particular, flexural strength.

Since the (meth)acrylate monomer not having an acid group, having two or less of hydroxyl groups and/or amino groups, and having a molecular weight of 160 or more, that is components of the first paste and the second paste in the dental cement in accordance with the present invention, has few hydrophilic group portion relatively to the molecular weight, the cured cement after the polymerization is difficult to absorb water and, as a result, hygroscopic expansion hardly occurs, therefore, it can be used to a ceramic crown type prosthesis having low strength. Further, since the cured cement after the polymerization is more stable in water, the solubility in water can also be decreased. Moreover, there is an effect to enhance the mechanical strength, in particular, flexural strength of the cured cement. When the molecular weight is less than 160, the hydrophilic group portion is large relatively to the molecular weight, thereby leading the cured cement after the polymerization readily to absorb water and expand, which is inadequate. Also, when 3 or more in total of the hydroxyl groups and/or amino groups are present, even though the molecular weight is 160 or more, the rate of hydrophilic groups is increased and the hygroscopic expansion of the cured cement after the polymerization becomes large, which is inadequate.

When the mixing quantity of the (meth)acrylate monomer not having an acid group, having two or less of hydroxyl groups and/or amino groups, and having a molecular weight of 160 or more is less than 5 % by weight in the first paste, or less than 10 % by weight in the second paste, the above effects can not be achieved. When over 55 % by weight thereof is mixed in the first paste or over 75 % by weight thereof is mixed in the second paste, the adhesiveness to tooth structure is deteriorated.

For the (meth)acrylate monomer not having an acid group, having two or less of hydroxyl groups and/or amino groups and having a molecular weight of 160 or more, many of monomers that have been conventionally used in dentistry may be employed, and for example, benzyl(meth)acrylate; 2,2-bis[(meth)acryloxy phenyl]propane; 2,2-bis[4-(meth)acryloxydiethoxy phenyl]propane; 2,2-bis[4-(meth)acryloxypolyethoxy phenyl]propane; ethylene glycol di(meth)acrylate; diethylene glycol di(meth)acrylate; triethylene glycol di(meth)acrylate; butylene glycol di(meth)acrylate; neopentyl glycol di(meth)acrylate; 1,3-butanediol di(meth)acrylate; 1,4-butanediol di(meth)acrylate; 1,6-hexanediol di(meth)acrylate; trimethylol propane tri(meth)acrylate; pentaerythritol tri(meth)acrylate; trimethylolmethane tri(meth)acrylate; pentaerythritol tetra(meth)acrylate; 2-hydroxyethyl (meth)acrylate; 2-hydroxypropyl (meth)acrylate; 2-hydroxy-1,3-di(meth)acryloxypropane; 1,2-dihydroxy-3-(meth)acryloxypropane; and 2,2-bis[4-{2-hydroxy-3-(meth)acryloxypropoxy}phenyl]propane can be included. For the polymerizable monomer having urethane bond and no acid group in the molecule, for example, di-2-(meth)acryloxyethyl-2,2,4-trimethylhexamethylene dicarbamate can be included.

For the filler having an average particle size from 0.05 to 20 µm and being inactive to the (meth)acrylate monomer having an acid group, powders such as silicon dioxide, metal oxides and a various kind of glass powders may be used and mixed in the first paste in the mixing quantity of 10 to 80 % by weight. When the mixing quantity thereof is less than 10 % by weight, the viscosity of the first paste is so low that the viscosity of the paste after the mixing with the second paste will be too low. Also, there is the possibility of separation of the paste into the solid portion and the solution portion during the storing. When the mixing quantity of the filler is over 80 % by weight, the viscosity of the first paste is so high that the first paste will be difficult to extrude from a syringe package. Also, the viscosity of the paste after mixing with the second pastes will be too high, which is inadequate.

It is the reason why the average particle size of the filler in the first paste must be 0.05 to 20 µm that when it is less than 0.05 µm, the viscosity of the paste is too high, while when it is over 20 µ m, the film thickness of the cement between a tooth surface and a dental prosthesis becomes large which makes the fitness with the dental prosthesis deteriorate.

For the amine compound as the polymerization accelerator for the polymerizing catalyst in the second paste to be mixed into the first paste in the dental cement in accordance with the present invention, aromatic tertiary amines, aliphatic tertiary amines, and the like are effective. Specifically, for examples of the amine compounds, N,N-dimethyl-p-toluidine; N,N-diethyl-p-toluidine; N,N-dimethylaniline; N,N-bis(2-hydroxyethyl)-p-toluidine; N,N-dimethylaminoethylmethacrylate; triethanolamine; methyl 4-dimethylaminobenzoate; ethyl 4-dimethylaminobenzoate; isoamyl 4-dimethylaminobenzoate; triethylamine; N-ethyldiethanolamine; and triethanolamine can be included. These amine compounds may be used solely or as a mixture of two or more of them.

This amine compound is required to be contained in the first paste in the quantity ranging from 0.01 to 5 % by weight. When the quantity thereof is less than 0.01 % by weight, the function thereof as the polymerization accelerator for the polymerizing catalyst in the second paste is not sufficient. When it is over 5 % by weight, the cured cement may be discolored even though the effect is hardly increased.

The second paste in the dental cement in accordance with the present invention provides a filler component of the dental cement together with the filler in the first paste. The average particle size of the filler in the second paste is in the range from 0.05 to 20 µm. The filler may be any powder of silicon dioxide, metal oxides and other various glass powders which are inactive to the (meth)acrylate monomer having an acid group in the first paste, or may be metal oxide powder that can cause cement reaction with the (meth)acrylate monomer having an acid group in the first paste (acid-base reaction) under the presence of water to cure, and is used for fluoroaluminosilicate glass powder, dental zinc phosphoric acid cement powder or dental carboxylate cement powder. Those may be mixed in the second paste in the quantity ranging from 20 to 85 % by weight. When the quantity thereof is less than 20 % by weight, the viscosity of the second paste is so low that the viscosity of the paste after mixing with the first paste will be unacceptably low. Also, there is the possibility of the separation of the solid portion and the liquid portion in the paste during the storage thereof. On the other hand, when the quantity is over 85 % by weight, the viscosity of the second paste is so high that the second paste will be difficult to extrude from a syringe package. Also, the viscosity of the paste after mixing with the first paste will be too high, which is inadequate.

It is the reason why the average particle size of the filler in the second paste must be in the range from 0.05 to 20 µm that when it is less than 0.05 µm, the viscosity of the paste is too high, while when it is over 20 µm, the film thickness of the cement between the tooth surface and the dental prosthesis becomes large which makes the fitness with the dental prosthesis deteriorate.

Said fluoroaluminosilicate glass powder is the glass powder that has been conventionally used for dental glass ionomer cement and preferably comprises Al³⁺, Si⁴⁺, F⁻ and O²⁻ as a main component and further include Sr²⁺ and/or Ca²⁺. Moreover, it is preferred that the fuluoroaluminosilicate glass powder comprises 10 to 21 % by weight of Al³⁺, 9 to 24 % by weight of Si⁴⁺, 1 to 20 % by weight of F⁻ and 10 to 34 % by weight of the total of Sr²⁺ and Ca²⁺ based on the total weight of the glass. The ratios of these main components provides much influence on the operation ability or physical properties such as the rate of curing, the resulting mechanical strength, and solubility when these components cause the cement reaction with the (meth)acrylate monomer having an acid group in the first paste under the presence of water (acid-base reaction). When the ratio of Al³⁺ is less than 10 % by weight, the curing rate is slow and the strength tends to be low. When the ratio of Al³⁺ is over 21 % by weight, production of glass is difficult and the transparency tends to be reduced to deteriorate aesthetic appearance. When the ratio of Si⁴⁺ is less than 9 % by weight, production of glass tends to also be difficult. When the ratio of Si⁴⁺ is over 24 % by weight, the curing rate tends to be slow and the mechanical strength also tends to be reduced to cause problem in the durability. When the ratio of F⁻ is less than 1 % by weight, the working time from mixing the first paste and the second paste is so short that a use operation tends to be difficult. When the ratio of F⁻ is over 20 % by weight, the setting time will be longer as well as the solubility in water will be larger to cause the durability deteriorated. When the total quantity of Sr²⁺ and Ca²⁺ is less than 10 % by weight, the sharpness of setting may not be attained and the setting time tends to be long. Further, production of glass tends to be difficult in this case. When the total quantity of Sr²⁺ and Ca²⁺ is over 34 % by weight, the working time is short and the setting time is short to cause the tendency of the difficulty of the actual use. In this case, the solubility in water is so large that the durability tends to be decreased. The fluoroaluminosilicate glass used in the present invention can be prepared by any conventional glass manufacturing processes.

Furthermore, the dental zinc phosphoric acid powder or the dental carboxylate cement powder is metal oxides powder including zinc oxide as a main component. They can be typically prepared by mixing 70 to 90 % by weight of zinc oxide with 10 to 30 % by weight of the metal oxide such as magnesium oxide, sintering the mixture at the temperature of 700 degrees C or above, then, cooling it and milling by a ball mill and the like. The other metal oxides than magnesium oxide, for example, may include strontium oxide, silicon dioxide, ferric oxide, yttrium oxide.

For the filler to be mixed in the first paste and the second paste, the filler to which silane treatment is applied by a conventional process may be used.

The organic aromatic compound having at least one -SO₂- group and the peroxide which are the polymerizing catalyst in the components in the second paste in the dental cement in accordance with the present invention, acts as the polymerizing catalyst for polymerizing the (meth)acrylate monomer having an acid group with the (meth)acrylate monomer not having an acid group, having two or less of the hydroxyl groups and/or amino groups and having a molecular weight of 160 or more by the action of the amine compound in the first paste as the polymerization accelerator, in particular, because of the presence of the organic aromatic compound having at least one -SO₂- group, the polymerization of the (meth)acrylate monomers can be enhanced. The organic aromatic compound having at least one -SO₂- group is aromatic sulfinic acids or the salts thereof, or aromatic sulfonyl compounds. For example thereof, sodium p-toluenesulfinic acid; lithium p-toluenesulfinic acid; benzenesulfinic acid; sodium benzenesulfinic acid; p-toluenesulfonyl chloride; p-toluenesulfonyl fluoride; o-toluenesulfonyl isocyanate; p-toluenesulfonyl hydrazide; p-toluenesulfonamide; p-toluenesulfonyl imidazol; p-toluenesulfonyl cyanide; 2-(p-toluenesulfonyl) acetophenone; p-toluenesulfonyl-N-diethylamide; α-N,α-toluenesulfonyl-N-arginine; α -N,p-toluenesulfonyl-L-arginine methyl ester; p-toluenesulfonyl methyl isocyanate; p-toluenesulfonyl-N-methyl-N-nitrosamide; N-(p-toluenesulfonyl)-L-phenylalanine; N-p-toluenesulfonyl-L-phenylalanine chloride; p-toluenesulfonyl acetonitrile; 2-(p-toluene-sulfonyl)acetophenone; toluene-3,4-disulfonyl chloride; benzene-sulfoneamide; benzenesulfohydroxamic acid; benzenesulfonyl chloride; benzenesulfonyl isocyanate; benzenesulfoneanilide; sodium benzenesulfone chloramide; benzene-sulfone-dichloramide; benzenesulfonyl hydrazide; benzene-sulfonyl-N-methylamide; 2-phenylsulfonyl acetophenone; diaminodiphenyl sulfone; 4,4'-sulfonyl diphenol; sulfapyridine; sulfa aerosol; sulfame-thyzol; ethylbenzenesulfonyl chloride; nitrobenzene sulfonyl chloride; and nitrobenzene sulfonyl fluoride may be included. Also, the organic aromatic compounds having at least one -SO₂- may be a hydrate salt.

For the peroxides, examples thereof include potassium peroxodisulfate; sodium peroxodisulfate; ammonium peroxodisulfate; benzoyl peroxide; 4,4'-dichlorobenzoyl peroxide; 2,4-dichlorobenzoyl peroxide; and dilauroyl peroxide. Among them, potassium peroxodisulfate or benzoyl peroxide are specially preferred. Those may be used by mixing one or 2 or more thereof.

The mixing ratio of the organic aromatic compound having at least one -SO₂- group and the peroxide is totally in the range from 0.01 to 10 % by weight based on the second paste. When it is less than 0.01 % by weight, ability as the polymerizing catalyst is not sufficient and the polymerization of the (meth)acrylate monomer having an acid group with the (meth)acrylate monomer not having an acid group, having two or less of hydroxyl groups and/or amino groups and having a molecular weight of 160 or more will be heterogeneous. When it is mixed in the amount of over 10 % by weight, the cured cement may be discolored even though the effect is hardly increased.

The mixing ratio of the first paste and the second paste of the dental cement in accordance with the present invention is 0.25 to 5 of the second paste with respect to 1 of the first paste by weight. When the ratio is less than 0.25, the mechanical strength of the dental cement after curing tends to be decreased. When the ratio is over 5, the adhesiveness to tooth structure tends to be deteriorated. In particular, the ratio in the range from 0.8 to 3 of the second paste with respect to 1 of the first paste by weight is preferred in view of mixing operation and the viscosity of the paste after the mixing.

When the filler having the average particular size in the range from 0.05 to 20 µm in the second paste is metal oxide powder that causes the cement reaction with the (meth)acrylate monomer having an acid group in the first paste under the presence of water, and is used in fluoroaluminosilicate glass powder, dental zinc phosphoric acid cement powder or dental carboxylate cement powder, and water is mixed into the second paste, it is preferred that the ratio of the water in the mixture of the first paste and the second paste is in the range of 3 to 10 % by weight in order to surely cause the cement reaction. Therefore, the mixing ratio of the first paste and the second paste may be determined by considering the ratio of the water mixed in the second paste.

In the dental cement in accordance with the present invention, it is preferred to employ a thickening agent for the purpose of attaining the paste of the first and the second pastes having high operability. The thickening agent used in the present invention is in the quantity that does not affect to the physical properties of the cured cement, specifically in the range from 0.1 to 10 % by weight, preferably in the range from 0.5 to 5 % by weight. When the quantity of the thickening agent is less than 0.1 % by weight, the effect thereby is hardly obtained, while when it is over 10 % by weight, the adhesiveness thereof to tooth structure tends to be deteriorated.

For such a thickening agent used in the present invention, any one of inorganic and organic ones may be employed. For example, inorganic thickening agents such as fumed silica having the average particle size in the range from 5 to 40 nm, and organic thickening agents such as calcium carboxymethyl cellulose, sodium carboxymethyl cellulose, starch, sodium starch glycolate, sodium starch phosphate ester, methyl cellulose, sodium polyacrylate, alginic acid, sodium alginate, propylene glycol alginate ester, casein, sodium casein, polyethylene glycol, ethyl cellulose, hydroxyethyl cellulose, gluten, locust bean gum, and gelatin can be included. These thickening agents may also be used by mixing two or more thereof.

In the dental cement in accordance with the present invention, 0.01 to 3 % by weight of a photo polymerizing catalyst can be used in any one of the above mentioned pastes in addition to the reaction by chemical polymerization. By using the chemical polymerizing catalyst together with the photo polymerizing catalyst, rapid photo polymerization reaction by irradiating visible light is applied in addition to the fast polymerization reaction of the polymerizing monomers by the chemical polymerization. In this case, methods for separately using the photo polymerization and the chemical polymerization as desired, for example, photo polymerization for temporary curing of excess cement after luting, photo polymerization for curing at the time of luting of a semitransparent inlay or crown made of ceramic or resin, chemical polymerization for luting of a metallic opaque inlay or crown can be employed, therefore, the further enlarged applications thereof can be expected.

When the photo polymerizing catalyst is employed, by irradiating an active light such as ultraviolet and visible light, the polymerization reaction of the polymerizing monomers can be achieved. For the light source therefor, super high pressure, high pressure, middle pressure and low pressure mercury vapor lamps, a chemical lamp, carbon arc lamp, metal hydride lamp, fluorescence lamp, tungsten lamp, xenon lamp, and argon ion laser may be used.

Of course, a polymerization inhibitor, an ultraviolet absorber, an antibacterial agent, a pigment, a stabilizer and the like which have typically been used may be properly mixed in the dental cement in accordance with the present invention if desired.

The present invention will now be described in more detail below with reference to the following embodiments.

### Filler to be mixed in the first paste

SiO₂ filler: silicon dioxide having the average particle size of about 2 µm (Trade name: Fuse Rex X, manufactured by Tatsumori K.K.)

### Filler to be mixed in the second paste, which is inactive to the (meth)acrylate monomer having an acid group in the first paste

SiO₂ filler: silicon dioxide having the average particle size of about 5 µm (Trade name: Crystalise VX-S2, manufactured by Tatsumori K.K.)

### Filler to be mixed in the second paste, which is active to the (meth)acrylate monomer having an acid group in the first paste

### [Preparation of fluoroaluminosilicate glass powder]

The components of fluoroaluminosilicate glass powders I, II and III are shown in Table 1.

**Table 1**

| | Fluoroaluminosilicate glass powder | | |
|---|---|---|---|
| | I | II | III |
| Aluminum oxide (g) | 21 | 23 | 22 |
| Silicic acid anhydride (g) | 44 | 41 | 43 |
| Calcium fluoride (g) | 12 | 10 | 12 |
| Calcium phosphate (g) | 14 | 13 | 15 |
| Strontium carbonate (g) | 9 | 13 | 8 |

For each of the fluoroaluminosilicate glass powders I and III, the raw materials were sufficiently mixed and placed in a high temperature electric furnace at 1,200 degrees C for 5 hours to melt the glass component. After the melt, the mixture was cooled, ground by a ball mill for 10 hours and passed through a 200 mesh (ASTM) sieve. Then, 1 g of γ -methacryloxy propyltrimethoxysilane was added to 100 g of the resulting powder together with 9 g of ethanol to conduct dry silane coupling according to the conventional process, thereby preparing fluoroaluminosilicate glass powder. For the fluoroaluminosilicate glass powder II, the same operation for preparing the same was conducted by the same process of the fluoroaluminosilicate glass powders I and III except that the mixture of the raw material was melted at 1,100 degrees C to prepare the fluoroaluminosilicate glass powder.

### [Preparation of the metal oxide powder other than thefluoroaluminosilicate glass powder]

The components of metal oxide powders I and II used in the dental zinc phosphate cement and the dental carboxylate cement powder are shown in Table 2.

**Table 2**

| | Metal oxide powder | |
|---|---|---|
| | I | II |
| Zinc oxide (g) | 88 | 80 |
| Magnesium oxide (g) | 12 | 18 |
| Strontium oxide (g) | - | 2 |

For metal oxide powder I, the raw materials were sufficiently mixed, placed in a high temperature electric furnace at 1,000 degrees C, maintained for 5 hours and sintered. After sintering, the resulting product was ground by using a ball mill for 10 hours and passed through a 200 mesh (ASTM) sieve to prepare a metal oxide powder having zinc oxide as a main component. Similarly, for metal oxide powder II, the mixture was sintered at 900 degrees C and subjected to the same processes of metal oxide powder I to prepare a metal oxide powder having zinc oxide as a main component.

### [Preparation of the first paste and the second paste]

The components of the first paste and the second paste used in each of Examples and Comparative Examples are shown in Table 3.

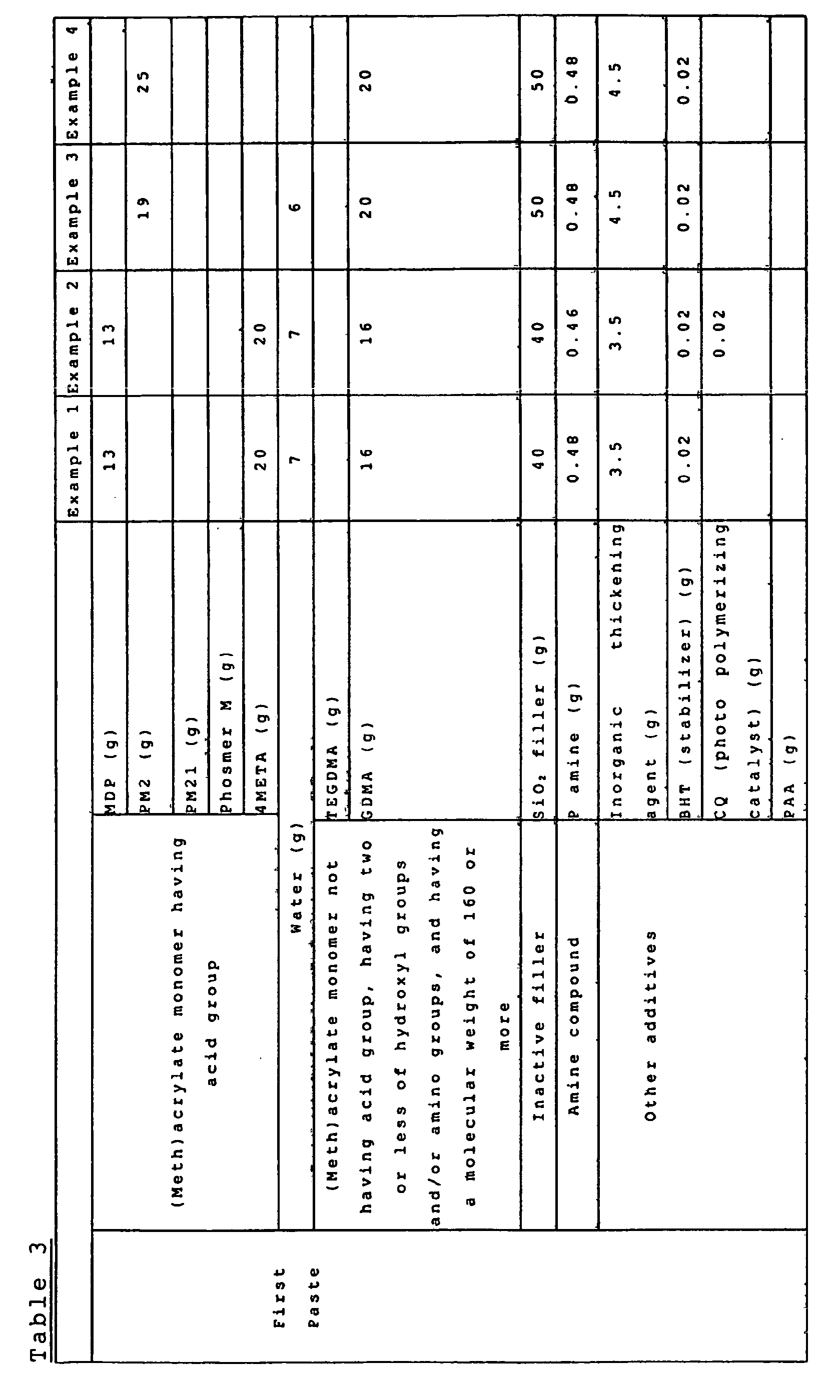

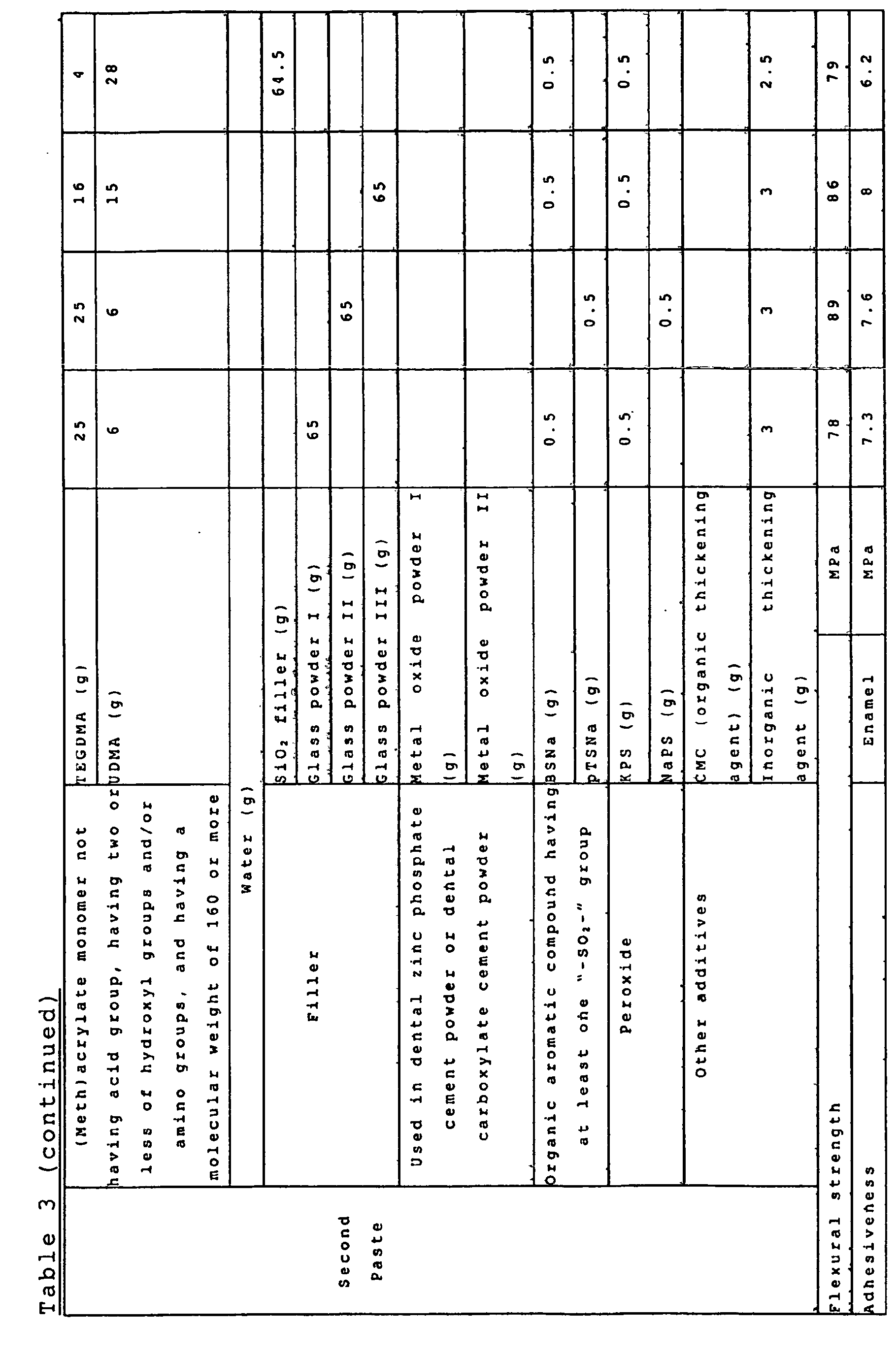

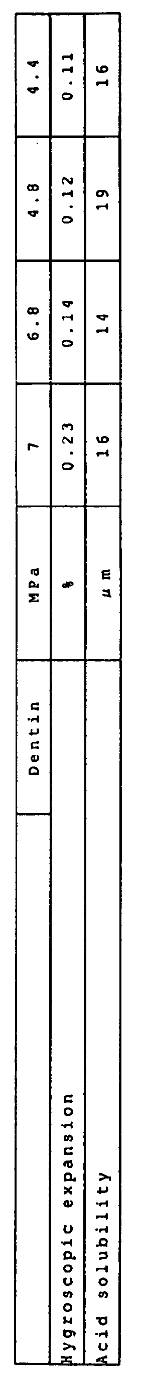

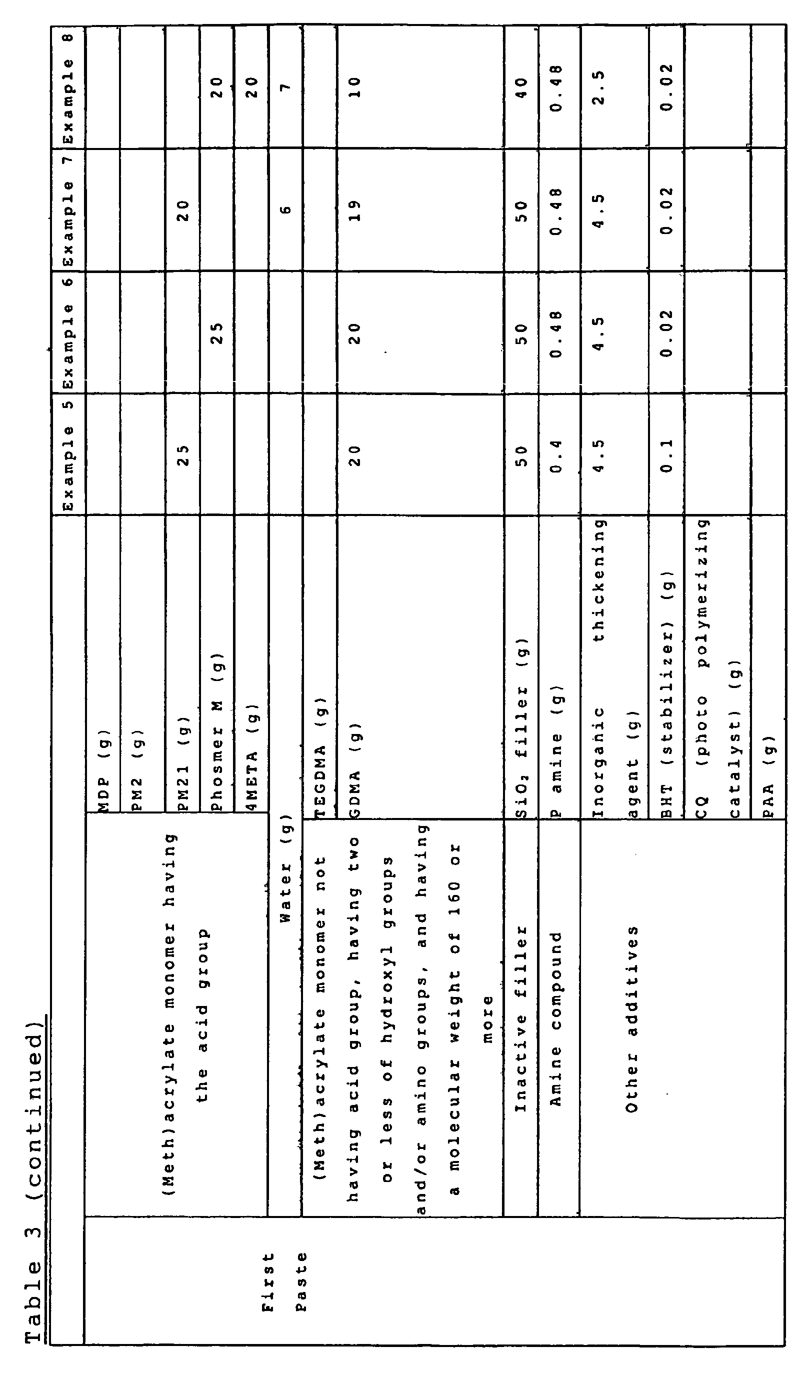

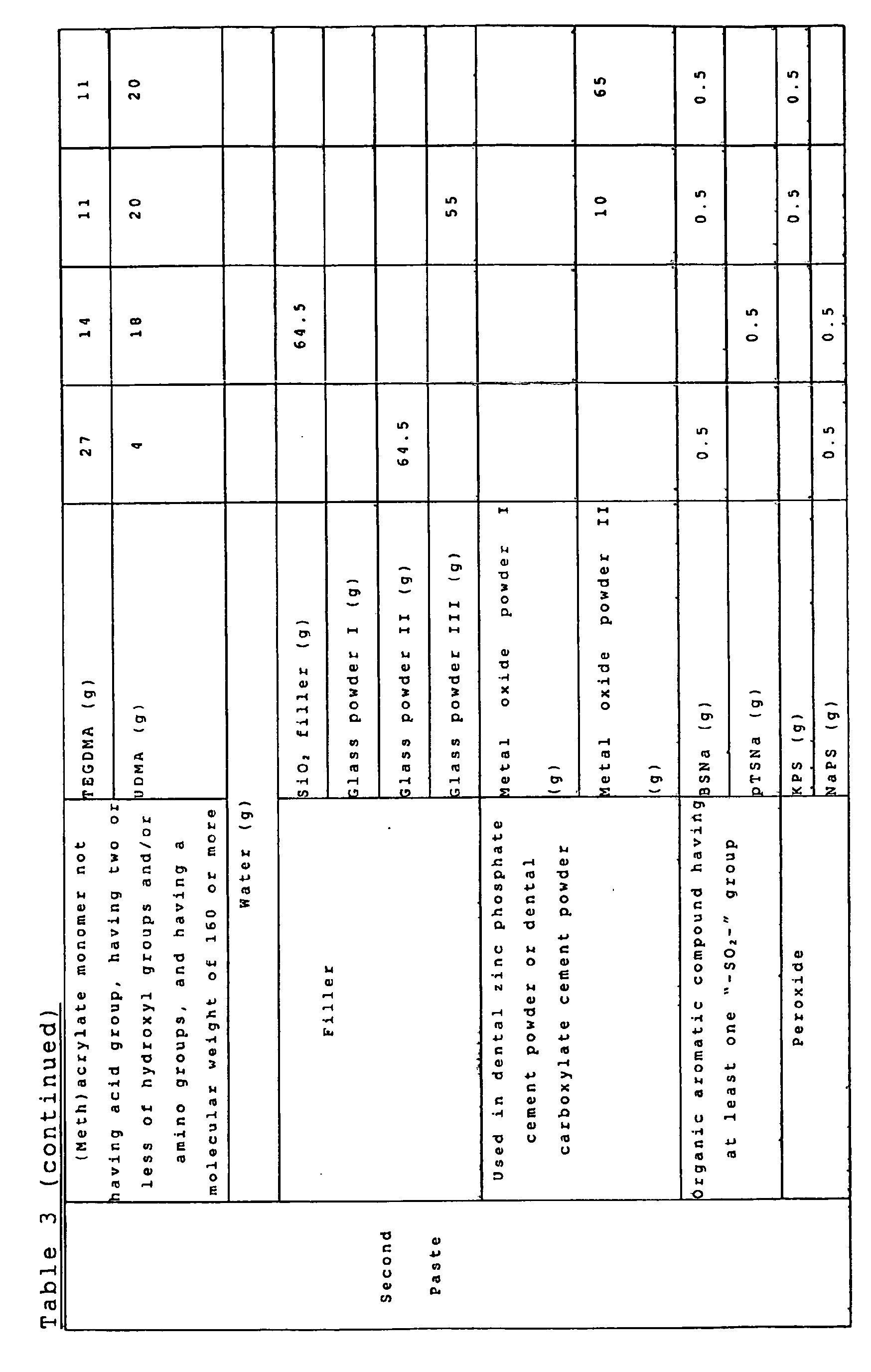

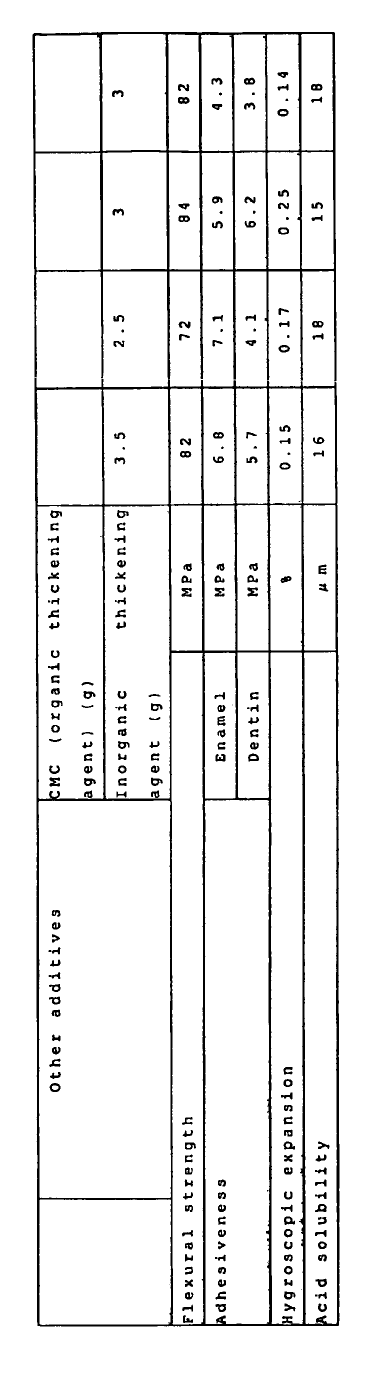

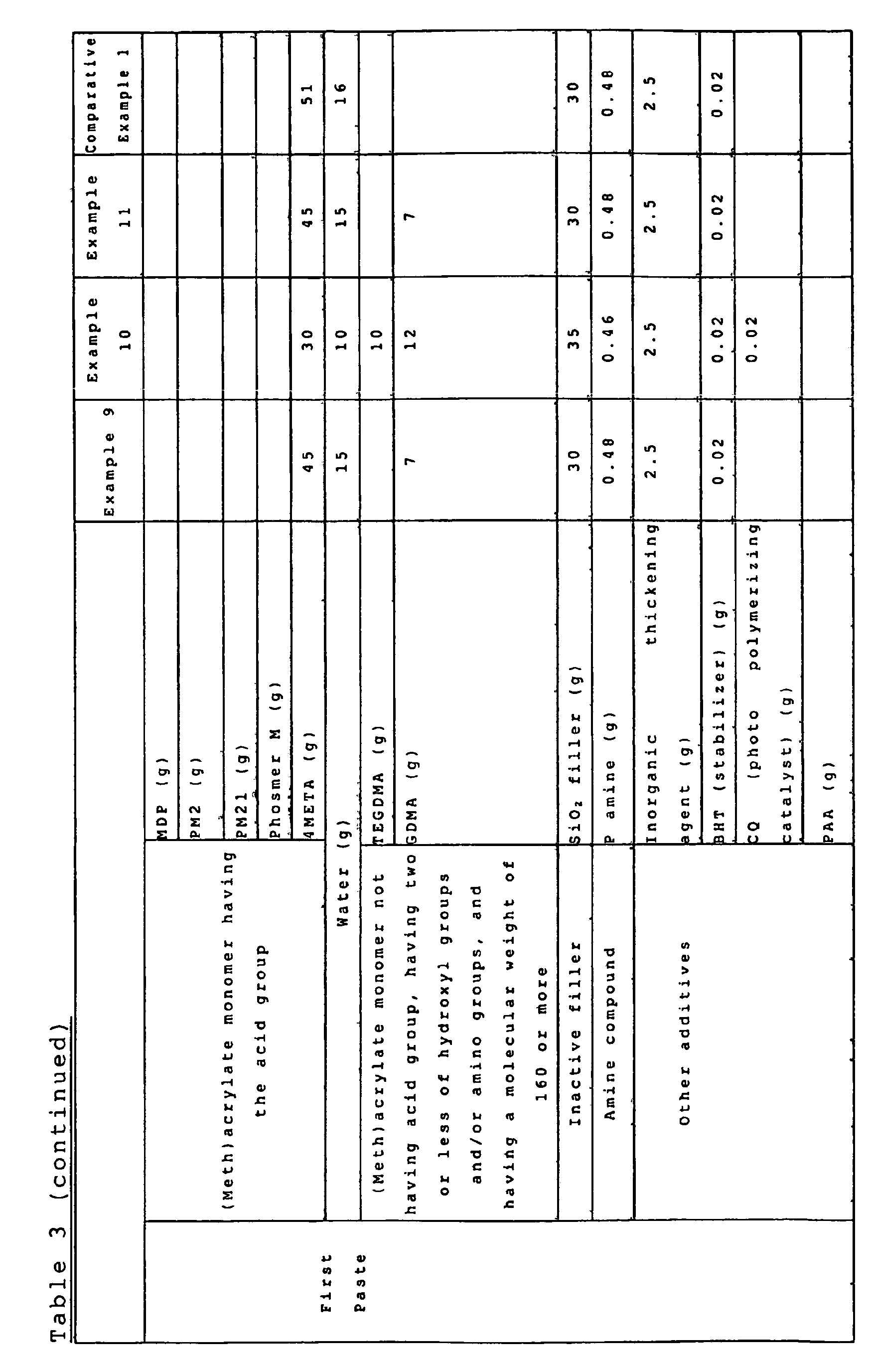

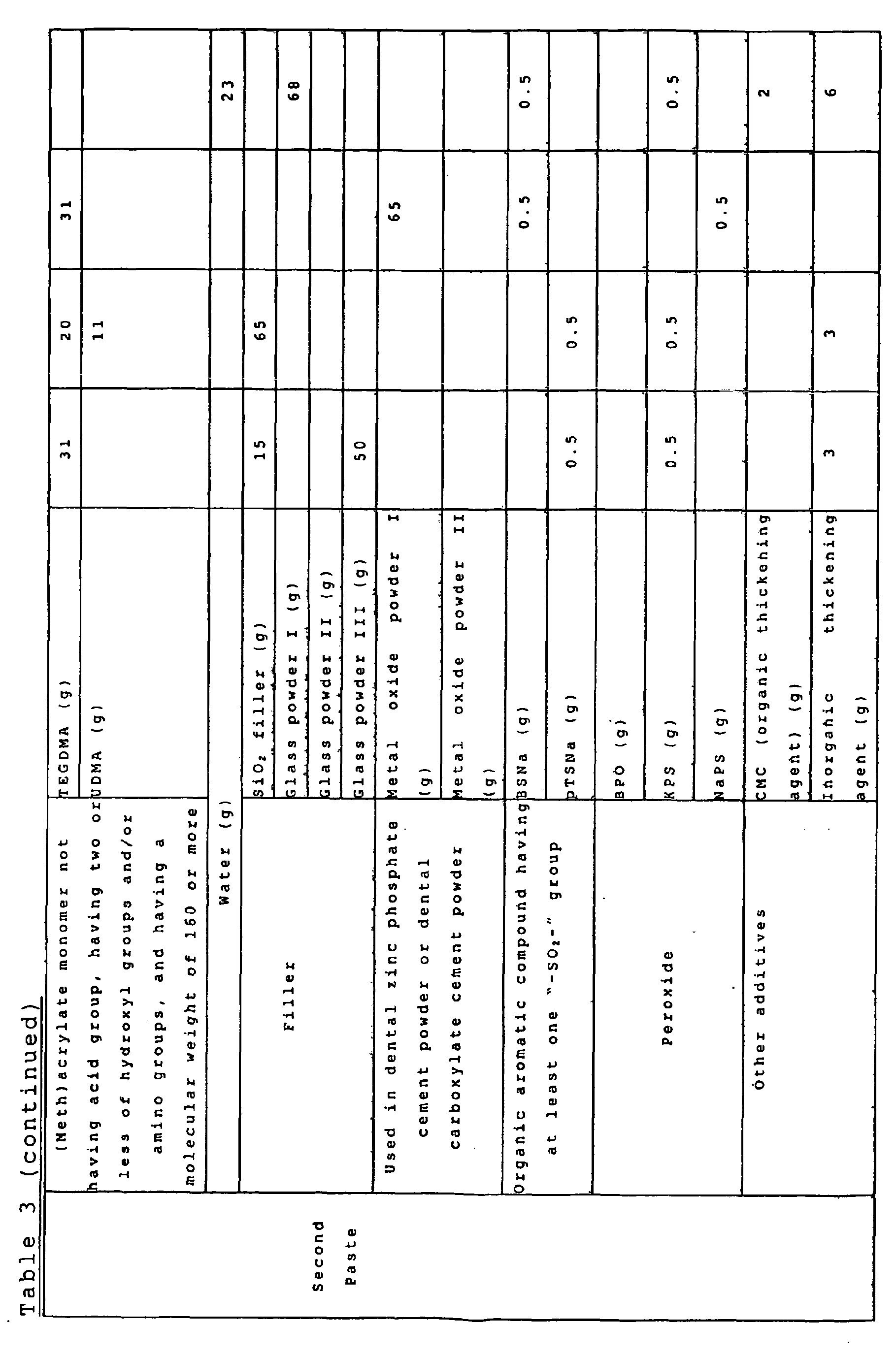

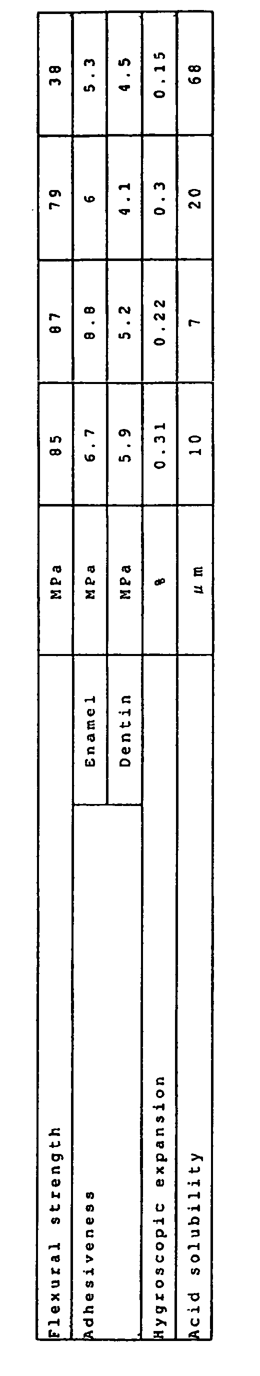

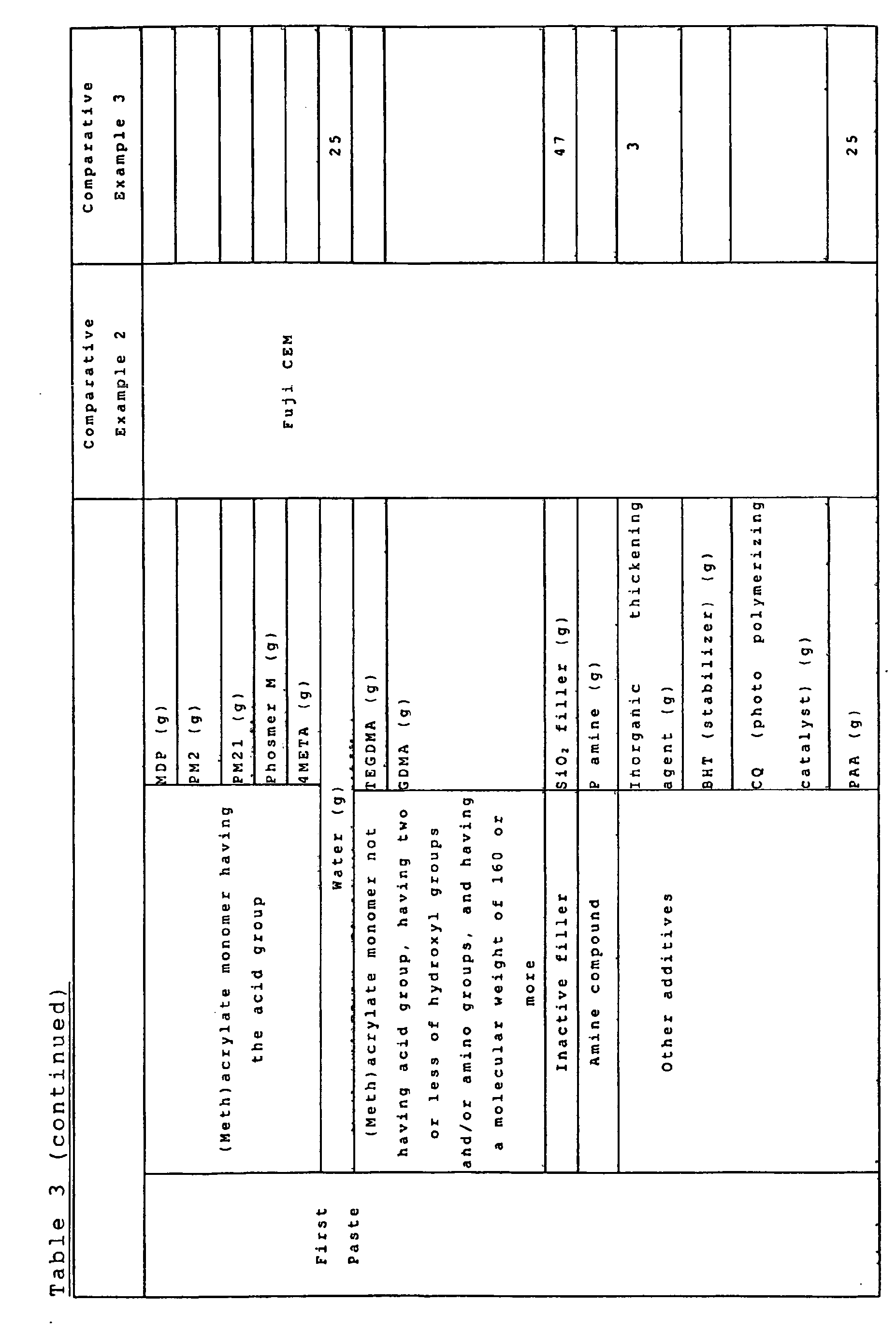

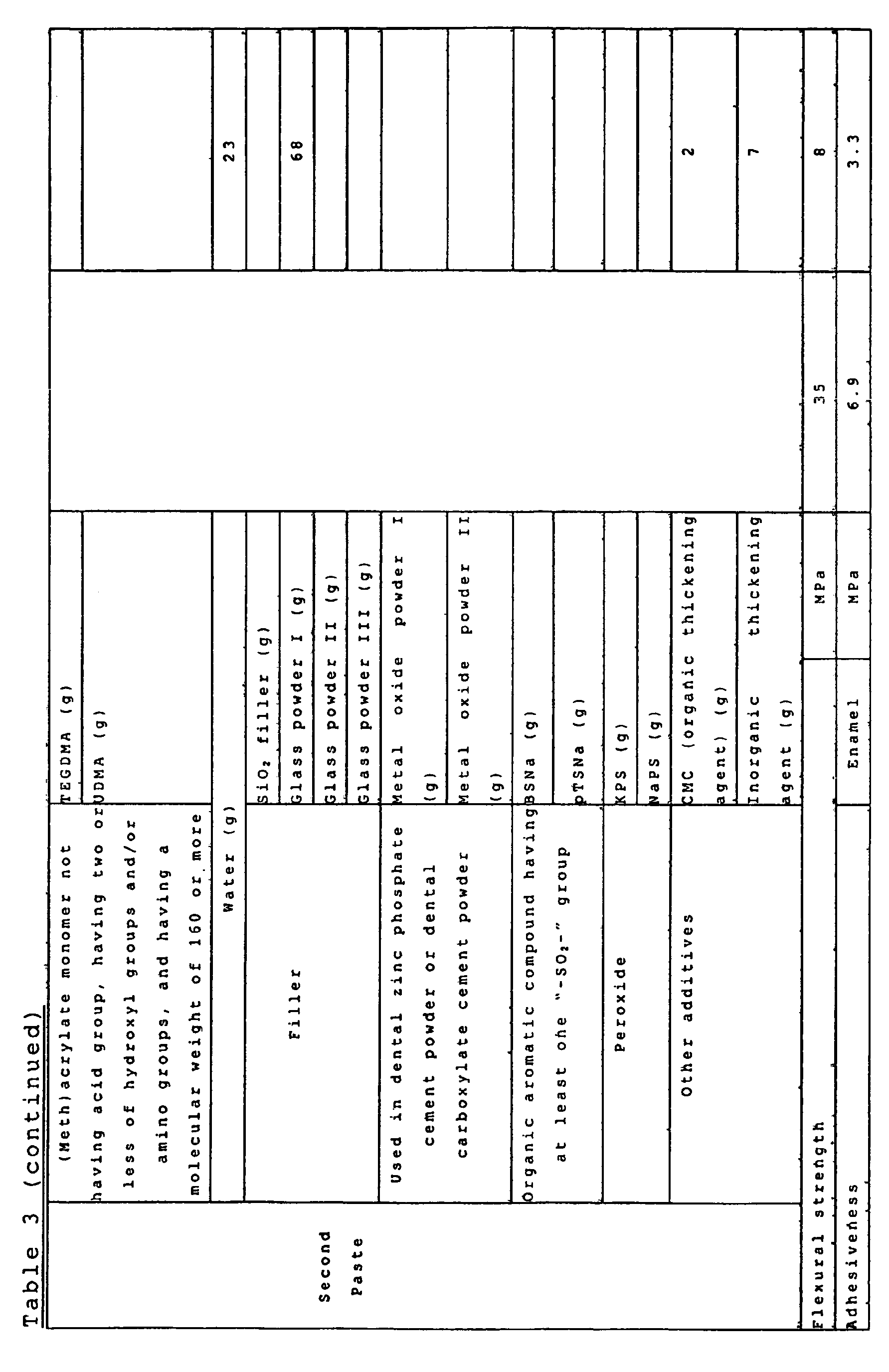

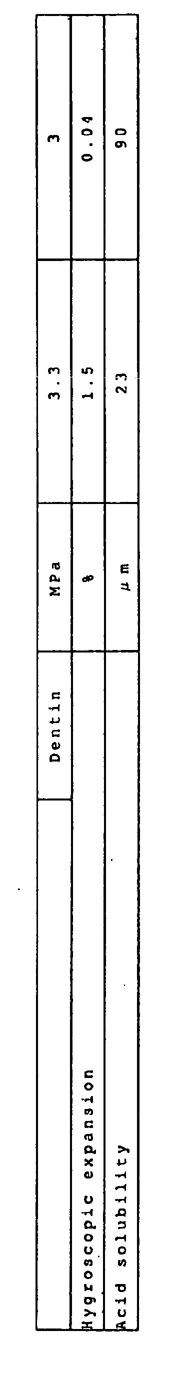

Each abbreviation in Table 3 is as follows.

### (Meth)acrylate monomer having an acid group

MDP: 10-methacryloyloxy decyldihydrogen phosphate
PM2: bismethacryloyloxy ethylphosphate
PM21: reaction product of 6-hexanolide addition polymer of 2-hydroxyethyl(meth)acrylate with anhydrous phosphoric acid
Phosmer M: acid phosphoxyethyl methacrylate 4META: 4-methacryloxyethyl trimellitic acid anhydride

### (Meth)acrylate monomers not having an acid group, having two or less of hydroxyl groups and/or amino groups and having a molecular weight of 160 or more

TEGDMA: triethyleneglycol dimethacrylate
GDMA: 2-hydroxy-1,3-dimethacryloxy propane
UDMA: di-2-methacryloxyethyl-2,2,4-trimethylhexamethylene dicarbamate

### Amine compound

P amine: N,N-bis(2-hydroxyethyl)-p-toluidine

### Organic aromatic compounds having at least one -SO₂-group

BSNa: dehydrate sodium benzenesulfinate
pTSNa: tetrahydrate sodium p-toluenesulfinate

### Peroxide

BPO: benzoylperoxide
KPS: pottasium peroxodisulfate
NaPS: sodium peroxodisulfate

### Other additives

Inorganic thickening agent: fumed silica having the average particle size of about 30 nm
CMC(organic thickening agent): calcium carboxymethylcellulose
PAA: polyacrylic acid (weight average molecular weight: about 20,000)
CQ (photo polymerizing catalyst): camphor quinone
BHT (stabilizer): 2,6-di-tert-butyl-p-cresol

### Metal oxide powder

Glass powder I: fluoroaluminosilicate glass powder I
Glass powder II: fluoroaluminosilicate glass powder II
Glass powder III: fluoroaluminosilicate glass powder III
Metal oxide powder I: metal oxide powder I containing zinc oxide as a main component
Metal oxide powder II: metal oxide powder II containing zinc oxide as a main component

### [Test of tensile bond strength]

The surface of a bovine anterior tooth was polished by a waterproof abrasive paper #600 to expose its enamel and dentin to obtain a surface to be adhered. The area of the surface to be adhered was defined by a resin masking tape with a hole having a diameter of 3 mm. Then, the mixed dental cement composition was put on the surface to be adhered and a stainless steel cylindrical rod, the surface of which had been previously polished by a waterproof abrasive paper #120 and subjected to sandblasting, was luted by hand pressure from the above thereof. Further, in case of the dental cement composition containing the photo polymerizing catalyst, an acrylic cylindrical rod to which the same treatment has been applied was used and the light irradiation was made from the front, the rear, the left and the right for 20 seconds each by using a dental visible lighting unit (product name; GC CO-BEE, manufactured by GC Corporation) after the pressure contacting. The specimen were left in a thermostatic vessel at 370 degrees C and 100 % relative humidity for one hour and, then, immersed in water at 37 degrees C for 23 hours. After that, the tensile bond strength for each sample was determined at a cross head speed of 1.0 mm/min. by a multi-functional tester (product name: Autograph, manufactured by Shimadzu Corporation).

### [Flexural strength]

The mixed dental cement composition was filled into an acrylic tube having an inner diameter of 3 mm and a length of 25 mm to obtain a cylindrical cured cement. Further, in case of the dental cement composition containing the photo polymerizing catalyst, the cylindrical cured cement was subjected to light irradiation from four directions each for 20 seconds by using a dental visible lighting unit (product name; GC CO-BEE, manufactured by GC Corporation). The specimens were immersed in distilled water at 37 degrees C for 96 hours, then, the flexural strength for each sample was determined by three-point flexural at a span of 20 mm and a cross head speed of 1.0 mm/min. by a multi-functional tester (product name: Autograph, manufactured by Shimadzu Corporation).

### [Hygroscopic expansion]

The mixed dental cement composition was filled into a metallic mold having a diameter of 4 mm and a height of 6 mm to obtain a cured cement specimen. Further, in case of the dental cement composition containing the photo polymerizing catalyst, the composition was filled into a metallic mold and pressed through a film, and subjected to irradiation of light from the height direction on both front and rear surfaces each for 20 seconds by using a dental visible lighting unit (product name; GC CO-BEE, manufactured by GC Corporation). After 24 hours, the specimens were taken out and the initial length in the height direction for each of them was measured. Then, the specimens were immersed in distilled water at 37 degrees C for 24 hours and the length in the height direction for each specimen was determined. The initial length was subtracted from the length in the height direction measured after immersing in the distilled water at 37 degrees C for 24 hours and the obtained value was divided by the initial length and multiplied by 100 to obtain a rate of hygroscopic expansion.

### [Acid solubility]

The test for acid solubility was performed to evaluate the rate of solubility of the dental cement composition. The dental cement composition after mixing was filled into a mold made of polymethyl methacrylate with a hole having a diameter of 5 mm and the depth of 2 mm and pressed through a film and, then, the resulting product was left in a thermostatic container at 37 degrees C and 100 % relative humidity for 24 hours. Further, in case of the dental cement composition containing the photo polymerizing catalyst, irradiation of light was carried out to the cement surface for 20 seconds by a dental visible lighting unit (product name; GC CO-BEE, manufactured by GC Corporation) after being filled into the mold and pressed through a film, followed by being left in the thermostatic vessel at 37 degrees C and 100 % relative humidity for 24 hours. Then, the surface of the cured cement being kept in the mold was subjected to polishing with the water proof abrasive paper #600 under pouring water to level the surface, and the initial length between the surface of the cured cement and the opposite side surface were measured. This specimen was immersed in lactic acid/sodium lactate buffer solution of 0.1 mol/L at 37 degrees C (pH 2.74) for 24 hours and the lengths were measured in the same manner to evaluate the decreased value.

### Examples 1 to 11

In each example, 1.5 g of the first paste and 1.0 g of the second paste were weighed and placed on a mixing paper and the first paste and the second paste were homogeneously mixed with the use of a spatula for 30 seconds. The results of tensile adhesive strength test, flexural test, hygroscopic expansion test and acid solubility test for the dental cements are shown in Table 3.

### Comparative Example 1

The dental glass ionomer cement to which the liquid agent comprising the (meth)acrylate monomers having an acid group and water was applied instead of polycarboxylic acid, 2-hydroxyethyl methacrylate and the like was used as a conventional dental cement for the cement of Comparative Example 1 shown in Table 3. By weighing 1.5 g of the first paste and 1.0 g of the second paste, placing them on a mixing paper, and conducting the mixing operation similarly to those of Examples 1 to 11 by the use of a spatula, the liquid agents and the powder agents were homogeneously mixed. The methods for tests were similar to those of the examples.

### Comparative Example 2

For the conventional resin modified paste dental glass ionomer cement, "Fuji CEM (manufactured by GC Corporation)" was used. By weighing with an exclusive use dispenser to the both pastes and placing them on a mixing paper, and conducting the mixing operation similarly to those of Examples 1 to 11 by the use of a spatula, both pastes were homogeneously mixed. The methods for tests were similar to those of the examples.

### Comparative Example 3

For the paste dental glass ionomer cement including no conventional resin component, the cement of Comparative Example 3 in Table 3 was used. 1.5 g of the first paste and 1.0 g of the second paste were weighed and placed on a mixing paper and similar mixing operation to those in Examples 1 to 11 was conducted to homogeneously mix them. The methods for tests were similar to those of the examples.

As shown in Table 3, dental cements of Examples 1 to 11 exhibited that they had large flexural strength, adhesiveness to tooth structure, small hygroscopic expansion, and low solubility, thus, it could be determined that they were excellent dental cements.

## Claims

1. A dental cement comprising:
a first paste containing:
5 to 75 % by weight of a (meth)acrylate monomer having an acid group,
5 to 55 % by weight of a (meth)acrylate monomer not having an acid group, having two or less of hydroxyl groups and/or amino groups and having a molecular weight of 160 or more,
10 to 80 % by weight of a filler being inactive to the (meth)acrylate monomer having an acid group and having an average particle size ranging from 0.05 to 20 µm, and
0.01 to 5 % by weight of an amine compound as a polymerization accelerator for a polymerization catalyst in a second paste described below; and
a second paste containing:
10 to 75 % by weight of a (meth)acrylate monomer not having an acid group, having two or less of hydroxyl groups and/or amino groups and having a molecular weight of 160 or more,
20 to 85 % by weight of a filler having an average particle size ranging from 0.05 to 20 µm, and
0.01 to 10 % by weight in total of an organic aromatic compound having at least one -SO₂- group and a peroxide as a polymerization catalyst for polymerizing the (meth)acrylate monomer having an acid group and the (meth)acrylate monomer not having an acid group, having two or less of hydroxyl groups and/or amino groups and having a molecular weight of 160 or more;
the dental cement being used by mixing the first and second pastes in a weight ratio of the second paste to the first paste in the range from 0.25 to 5.

2. A dental cement as claimed in claim 1, wherein 1 to 20 % by weight of water is further mixed in the first paste.

3. A dental cement as claimed in claim 1 or 2, wherein the cement includes a monomer having a carboxyl group as the (meth)acrylate monomer having an acid group.

4. A dental cement as claimed in any one of claims 1 to 3, wherein the filler having an average particule size of 0.05 to 20 µm in the second paste is a filler that is inactive to the (meth)acrylate monomer having an acid group.

5. A dental cement as claimed in any one of claims 1 to 3, wherein the filler having an average particle size of 0.05 to 20 µm in the first paste is a metal oxide powder used in fluoroaluminosilicate glass powder, dental zinc phosphate cement powder or dental carboxylate cement powder.

6. A dental cement as claimed in claim 5, wherein the ratio of water in the mixture of the first paste and the second paste is in the range from 3 to 10 % by weight.

7. A dental cement as claimed in any one of claims 1 to 6, wherein 0.1 to 10 % by weight of an inorganic thickening agent and/or an organic thickening agent having an average particle size ranging from 5 to 40 nm is further mixed in either one of the pastes.

8. A dental cement as claimed in any one of claims 1 to 7, wherein 0.01 to 3 % by weight of a photo polymerizing catalyst is further mixed in either one of the pastes for accelerating the polymerization of (meth)acrylate monomer having an acid group and (meth)acrylate monomer not having an acid group, having two or less of hydroxyl groups and/or amino groups and having a molecular weight of 160 or more.

## Patentansprüche

1. Dentalzement, umfassend:
eine erste Paste, enthaltend:
5 bis 75 Gew.-% eines (Meth)acrylatmonomers mit einer Säuregruppe,
5 bis 55 Gew.-% eines (Meth)acrylatmonomers, welches keine Säuregruppe aufweist, mit zwei oder weniger Hydroxylgruppen und/oder Aminogruppen und mit einem Molekulargewicht von 160 oder mehr,
10 bis 80 Gew.-% eines Füllmittels, welches gegenüber dem (Meth)acrylatmonomer mit einer Säuregruppe inaktiv ist, und mit einer mittleren Teilchengröße im Bereich von 0,05 bis 20 µm, und
0,01 bis 5 Gew.-% einer Aminverbindung als ein Polymerisationsbeschleuniger für einen Polymerisationskatalysator in einer nachfolgend beschriebenen zweiten Paste; und
eine zweite Paste, enthaltend :
10 bis 75 Gew.-% eines (Meth)acrylatmonomers, welches keine Säuregruppe aufweist, mit zwei oder weniger Hydroxylgruppen und/oder Aminogruppen und mit einem Molekulargewicht von 160 oder mehr,
20 bis 85 Gew.-% eines Füllmittels mit einer mittleren Teilchengröße im Bereich von 0,05 bis 20 µm, und
0,01 bis 10 Gew.-% in Summe einer organischen aromatischen Verbindung mit mindestens einer -SO₂-Gruppe und eines Peroxids als ein Polymerisationskatalysator zum Polymerisieren des (Meth)acrylatmonomers mit einer Säuregruppe und des (Meth)acrylatmonomers, welches keine Säuregruppe aufweist, mit zwei oder weniger Hydroxylgruppen und/oder Aminogruppen und mit einem Molekulargewicht von 160 oder mehr;
wobei der Dentalzement durch Mischen der ersten und zweiten Paste in einem Gewichtsverhältnis der zweiten Paste zu der ersten Paste in dem Bereich von 0,25 bis 5 verwendet wird.

2. Dentalzement nach Anspruch 1, wobei weiter 1 bis 20 Gew.-% Wasser in die erste Paste gemischt ist.

3. Dentalzement nach Anspruch 1 oder 2, wobei der Zement ein Monomer mit einer Carboxylgruppe als das (Meth)acrylatmonomer mit einer Säuregruppe einschließt.

4. Dentalzement nach einem der Ansprüche 1 bis 3, wobei das Füllmittel mit einer mittleren Teilchengröße von 0,05 bis 20 µm in der zweiten Paste ein Füllmittel ist, welches gegenüber dem (Meth)acrylatmonomer mit einer Säuregruppe inaktiv ist.

5. Dentalzement nach einem der Ansprüche 1 bis 3, wobei das Füllmittel mit einer mittleren Teilchengröße von 0,05 bis 20 µm in der ersten Paste ein Metalloxidpulver ist, verwendet in Fluoralumosilikatglaspulver, Dentalzinkphosphatzementpulver oder Dentalcarboxylatzementpulver.

6. Dentalzement nach Anspruch 5, wobei das Verhältnis von Wasser in dem Gemisch der ersten Paste und der zweiten Paste in dem Bereich von 3 bis 10 Gew.-% liegt.

7. Dentalzement nach einem der Ansprüche 1 bis 6, wobei 0,1 bis 10 Gew.-% eines anorganischen Verdickungsmittels und/oder eines organischen Verdikkungsmittels mit einer mittleren Teilchengröße im Bereich von 5 bis 40 nm weiter in eine der Pasten gemischt ist.

8. Dentalzement nach einem der Ansprüche 1 bis 7, wobei weiter 0,01 bis 3 Gew.-% eines Photopolymerisationskatalysators in eine der Pasten zum Beschleunigen der Polymerisation von (Meth)acrylatmonomer mit einer Säuregruppe und (Meth)acrylatmonomer, welches keine Säuregruppe aufweist, mit zwei oder weniger an Hydroxylgruppen und/oder Aminogruppen und mit einem Molekulargewicht von 160 oder mehr, gemischt ist.

## Revendications

1. Ciment dentaire comprenant :
une première pâte contenant :
5 à 75 % en poids d'un monomère de (méth)acrylate ayant un groupe acide,
5 à 55 % en poids d'un monomère de (méth)acrylate n'ayant pas de groupe acide, ayant deux groupes hydroxyle et/ou groupes amino ou moins et ayant une masse moléculaire de 160 ou plus,
10 à 80 % en poids d'une charge inactive au monomère de (méth)acrylate ayant un groupe acide et ayant une taille de particule moyenne allant de 0,05 à 20 µm, et
0,01 à 5 % en poids d'un composé amine comme un accélérateur de polymérisation pour un catalyseur de polymérisation dans une seconde pâte décrite ci-après ; et
une seconde pâte contenant :
10 à 75 % en poids d'un monomère de (méth)acrylate n'ayant pas de groupe acide, ayant deux groupes hydroxyle et/ou groupes amino ou moins et ayant un poids moléculaire de 160 ou plus,
20 à 85 % en poids d'une charge ayant une taille de particule moyenne allant de 0,05 à 20 µm, et
0,01 à 10 % en poids au total d'un composé aromatique organique ayant au moins un groupe -SO₂- et un peroxyde en tant que catalyseur de polymérisation pour polymériser le monomère de (méth)acrylate ayant un groupe acide et le monomère de (méth)acrylate n'ayant pas de groupe acide, ayant deux groupes hydroxyle et/ou groupes amino ou moins et ayant une masse moléculaire de 160 ou plus ;
le ciment dentaire étant utilisé en mélangeant les première et seconde pâtes dans un rapport en poids de la seconde pâte sur la première pâte dans la plage de 0,25 à 5.

2. Ciment dentaire selon la revendication 1, dans lequel 1 à 20 % en poids d'eau est en outre mélangé dans la première pâte.

3. Ciment dentaire selon la revendication 1 ou 2, dans lequel le ciment comprend un monomère ayant un groupe carboxyle en tant que monomère de (méth)acrylate ayant un groupe acide.

4. Ciment dentaire selon l'une quelconque des revendications 1 à 3, dans lequel la charge ayant une taille de particule moyenne de 0,05 à 20 µm dans la seconde pâte est une charge qui est inactive au monomère de (méth)acrylate ayant un groupe acide.

5. Ciment dentaire selon l'une quelconque des revendications 1 à 3, dans lequel la charge ayant une taille de particule moyenne de 0,05 à 20 µm dans la première pâte est une poudre d'oxyde métallique utilisée dans une poudre de verre de fluoroaluminosilicate, une poudre de ciment de phosphate de zinc dentaire ou une poudre de ciment de carboxylate dentaire.

6. Ciment dentaire selon la revendication 5, dans lequel le rapport de l'eau dans le mélange de la première pâte et de la seconde pâte est dans la plage de 3 à 10 % en poids.

7. Ciment dentaire selon l'une quelconque des revendications 1 à 6, dans lequel 0,1 à 10 % en poids d'un agent épaississant inorganique et/ou d'un agent épaississant organique ayant une taille de particule moyenne allant de 5 à 40 nm est en outre mélangé dans l'une ou l'autre des pâtes.

8. Ciment dentaire selon l'une quelconque des revendications 1 à 7, dans lequel 0,01 à 3 % en poids d'un catalyseur photopolymérisant est en outre mélangé dans l'une ou l'autre des pâtes pour accélérer la polymérisation du monomère de (méth)acrylate ayant un groupe acide et du monomère de (méth)acrylate n'ayant pas de groupe acide, ayant deux groupes hydroxyle et/ou groupes amino ou moins et ayant une masse moléculaire de 160 ou plus.
